# EUROPEAN PATENT SPECIFICATION

(11) **EP 2 892 340 B1**
(45) Date of publication and mention of the grant of the patent: **11.07.2018**
(21) Application number: 13748304.6
(22) Date of filing: 12.08.2013
(51) Int. Cl.: A01N 33/04, A01N 37/40, A01N 43/80, A01N 31/14, A01P 1/00

(54) **NOVEL COMPOSITIONS COMPRISING P-HYDROXYBENZYLAMINE**
NEUE ZUSAMMENSETZUNGEN ENTHALTEND P-HYDROXYBENZYLAMIN
NOUVELLES COMPOSITIONS COMPRENANT DE LA P-HYDROXYBENZYLAMINE

(30) Priority: 03.09.2012 EP 12182756
(43) Date of publication of application: 15.07.2015
(73) Proprietor: DSM IP Assets B.V., 6411 TE Heerlen (NL)
(72) Inventor: MENDROK-EDINGER, Christine, 4002 Basel (CH); VOEGELI, Rainer, 4002 Basel (CH); WIKSTROEM, Peter, 4002 Basel (CH)
(74) Representative: Berg, Katja
(86) International application number: PCT/EP2013/066813
(87) International publication number: WO 2014/032952

(56) References cited:
- US-A- 3 726 969
- SLAUGHTER J C ET AL: "The identification of p-hydroxybenzylamine in barley and malt", PHYTOCHEMISTRY, PERGAMON PRESS, GB, vol. 11, no. 1, 1 January 1972 (1972-01-01), pages 478-479, XP026609901, ISSN: 0031-9422, DOI: 10.1016/S0031-9422(00)90065-7 [retrieved on 1972-01-01]
- Masahiro Koyama ET AL: "Identification of Hydroxybenzylamines in Buckwheat Seeds (Fagopyrum esculentum Moench)", Agr. Biol. Chem., 1 January 1971 (1971-01-01), pages 1870-1879, XP055048150, Retrieved from the Internet: URL:https://www.jstage.jst.go.jp/article/b bb1961/35/12/35_12_1870/_pdf [retrieved on 2012-12-18]
- Miles D Houslay ET AL: "A Kinetic Evaluation of Monoamine Oxidase Activity in Rat Liver Mitochondrial Outer Membranes", Biochem. J., 1 January 1974 (1974-01-01), pages 645-652, XP055048143, Great Britain Retrieved from the Internet: URL:http://www.biochemj.org/bj/139/0645/13 90645.pdf [retrieved on 2012-12-18]
- TAO Y H ET AL: "Inhibition of GABA shunt enzymes' activity by 4-hydroxybenzaldehyde derivatives", BIOORGANIC & MEDICINAL CHEMISTRY LETTERS, PERGAMON, ELSEVIER SCIENCE, GB, vol. 16, no. 3, 1 February 2006 (2006-02-01), pages 592-595, XP027965839, ISSN: 0960-894X [retrieved on 2006-02-01]
- XINGLIANG LU ET AL: "Inactivation of mitochondrial monoamine oxidase B by methylthio-substituted benzylamines", BIOORGANIC & MEDICINAL CHEMISTRY, vol. 11, no. 20, 1 October 2003 (2003-10-01), pages 4423-4430, XP055048133, ISSN: 0968-0896, DOI: 10.1016/S0968-0896(03)00486-3
- ALESIANI D ET AL: "Antioxidant and antiproliferative activities of phytochemicals from Quince (Cydonia vulgaris) peels", FOOD CHEMISTRY, ELSEVIER LTD, NL, vol. 118, no. 2, 15 January 2010 (2010-01-15), pages 199-207, XP026497657, ISSN: 0308-8146, DOI: 10.1016/J.FOODCHEM.2009.04.098 [retrieved on 2009-05-03]
- Daniel V Santi ET AL: "Tyrosyl Transfer Ribonucleic Acid Synthetase from Escherichia coli B. Analysis of Tyrosine and Adenosine 5'-Triphosphate Binding Sites", Journal of Medicinal Chemistry, 1 March 1973 (1973-03-01), pages 273-280, XP055048139, U.S.A. Retrieved from the Internet: URL:http://pubs.acs.org/doi/pdf/10.1021/jm 00261a025 [retrieved on 2012-12-18]
- H S Bean: "Preservatives for pharmaceuticals", J. Soc. Cosmet. Chem., 1 January 1972 (1972-01-01), pages 703-720, XP055048193, Great Britain Retrieved from the Internet: URL:http://journal.scconline.org/pdf/cc197 2/cc023n11/p00703-p00720.pdf [retrieved on 2012-12-18]
- "Preservatives", , 1 April 2005 (2005-04-01), pages 1-22, XP055048187, Retrieved from the Internet: URL:http://www.custoblend.com/Combined Documents Preservatives.pdf [retrieved on 2012-12-18]
- LUNDOV M D ET AL: "Low-level efficacy of cosmetic preservatives", INTERNATIONAL JOURNAL OF COSMETIC SCIENCE, KLUWER ACADEMIC PUBLISHERS, DORDRECHT, NL, vol. 33, no. 2, 1 April 2011 (2011-04-01), pages 190-196, XP007917477, ISSN: 0142-5463, DOI: 10.1111/J.1468-2494.2010.00619.X [retrieved on 2011-01-27]
- N J Van Abbé ET AL: "The hygienic manufacture and preservation of toiletries and cosmetics", J. Soc. Cosmet. Chem., 1 January 1970 (1970-01-01), pages 719-800, XP055048298, Great Britain Retrieved from the Internet: URL:http://journal.scconline.org/pdf/cc197 0/cc021n11/p00719-p00800.pdf [retrieved on 2012-12-19]

## Description

The present invention relates to compositions comprising p-hydroxybenzylamine or a salt thereof and at least one preservative as well as to the use of p-hydroxybenzylamine or a salt thereof to boost the antimicrobial activity of at least one preservative.

To protect cosmetics against mold and bacteria, most cosmetic products currently on the market contain preservatives. While these preservatives protect against bacteria and fungi, studies have linked daily exposure to many of these substances to an increased risk of skin irritation, cancer and/ or endocrine problems. Thus, many cosmetic manufactures are searching for alternatives which allow reducing the amount of preservatives and don't appear to pose any health risks.

Lundov et al (International Journal of Cosmetic Science, 2011, 33, 190-196) discloses a composition comprising a preservative for use in cosmetic preparations. The publication furthermore discloses that the concentration of some of the most frequently used allergenic preservatives can be markedly lowered when they are combined with phenoxyethanol.

Surprisingly, it has been found that p-hydroxybenzylamine synergistically increases the antimicrobial activity of preservatives conventionally used in cosmetic applications thus allowing the reduction of the amount of preservatives used when formulating cosmetic compositions.

Thus, the present invention provides a composition comprising of p-hydroxybenzylamine or a salt thereof and at least one preservative selected from the group consisting of parabens, chloromethylisothiazolinone, methylisothiazolinone or phenoxyethanol as well as mixture thereof. Such compositions show an enhanced antimicrobial activity such as in particular an enhance anti-fungal and anti-bacterial efficiency.

In another embodiment, the invention relates to the use of p-hydroxybenzylamine or a salt thereof for increasing the antimicrobial activity of at least one preservative.

In a further embodiment the invention relates to a method of increasing the antimicrobial activity of at least one preservative, selected from the group consisting of parabens, chloromethylisothiazolinone, methylisothiazolinone or phenoxyethanol as well as mixtures thereofsaid method comprising the addition of p-hydroxybenzylamine or a salt thereof into a topical composition and observing or appreciating the result.

In addition, the invention relates to a non-therapeutic method for killing and/ or inhibiting growth of microbial cells, in particular fungal and/ or bacterial cells, said method comprising contacting said microbial cells with a mixture consisting of p-hydroxybenzylamine or a salt thereof and at least one preservative selected from the group consisting of parabens, chloromethylisothiazolinone, methylisothiazolinone or phenoxyethanol as well as mixtures thereof.

The ratio of p-hydroxybenzylamine or a salt thereof to the at least one preservative in the compositions according to the present invention depends on the preservative(s) used and can easily be determined by a person skilled in the art and is illustrated in the examples. Preferably an excess (in weight) of p-hydroxybenzylamine or a salt thereof is used, i.e. the ratio (w/w) of p-hydroxybenzylamine or a salt thereof to the total amount of preservative(s) is >1. In all embodiments according to the present invention preferably the ratio (w/w)of p-hydroxybenzylamine or a salt thereof to the (total) amount of preservative(s) is selected in the range of 2:1 to 100:1 such as in the range of 3:1 to 50:1.

The p-hydroxybenzylamine or a salt thereof and the preservative may be incorporated into the compositions according to the present invention either in the form of a pre-mix or may be added separately.

The amount of p-hydroxybenzylamine or a salt thereof in the compositions according to the present invention may easily be adapted by a person skilled in the art. Preferably in all embodiments according to the invention p-hydroxybenzylamine or a salt thereof is used in an amount of at least 0.0001 wt.-%. More preferably, p-hydroxybenzylamine or a salt thereof is used in an amount of about 0.001 to 10 wt.-%, in particular in an amount of about 0.005 wt.-% to 5 wt.-% such as in an amount of about 0.01 to 1 wt.-%, based on the total weight of the composition.

The total amount of preservative(s) in the compositions according to the present invention is not critical and can easily be adjusted by a person skilled in the art. Advantageously the (total) amount of the at least one preservative in the compositions according to the present invention is selected in the range of 0.001 to 5 wt.-% such as in particular in the range of 0.0.01 to 1 wt.-% such as most in particular in the range of 0.01 to 0.5 wt.-%, based on the total weight of the composition.

The term "antimicrobial activity" (or "antimicrobial effect") as used herein means a capability of killing and/ or inhibiting growth of microbial cells such as in particular bacteria and fungi.

p-Hydroxybenzylamine is also known as 4-hydroxybenzylamine respectively 4-(aminomethyl)-phenol [CAS 696-60-6] and is e.g. commercially available at Ugarit Chimie (Issy les Moulineux, France).

According to the present invention p-hydroxybenzylamine can also be employed in the form of a salt thereof. Suitable salts encompass e.g. salts obtainable with acids, such as e.g. with mineral acids such as hydrogen chloride, hydrogen bromide, sulphuric acid or phosphoric acid; with appropriate carboxylic acids, e.g. aliphatic mono- or dicarboxylic acids such as formic acid, acetic acid, trifluoroacetic acid, trichloroacetic acid, propionic acid, glycolic acid, succinic acid, fumaric acid, malonic acid, maleic acid, oxalic acid, phthalic acid, citric acid, lactic acid or tartaric acid; with aromatic carboxylic acids such as benzoic acid or salicylic acid; with aromatic-aliphatic carboxylic acids such as mandelic acid or cinnamic acid; with heteroaromatic carboxylic acids such as nicotinic acid; or with aliphatic or aromatic sulfonic acids such as methanesulfonic acid or toluenesulfonic acid. Particular suitable salts of p-hydroxybenzylamine for the purpose of the present invention are the hydrochloride [CAS 1004-23-5] or the hydrobromide [CAS 90430-14-1].

Preferably, in all embodiments of the present invention p-hydroxybenzylamine is used in the form of its hydrochloride. Nevertheless, even if incorporated as free amine, the actual form of p-hydroxybenzylamine in the compositions according to the present invention, i.e. its existence as free amine or a salt thereof may depend on the respective pH / mixture of a specific composition.

The term "paraben" as used herein refers to esters of para-hydroxybenzoic acid. Common parabens include methylparaben [CAS No. 99-76-3], ethylparaben [CAS No. 120-47-8], propylparaben [CAS No 94-13-3], butylparaben [CAS No. 94-26-8], heptylparaben [CAS1085-12-7], isobutylparaben [CAS No. 4247-02-3], isopropylparaben [CAS No. 4191-73-5] and benzylparaben [CAS No. 94-18-8] as well as salts thereof such as in particular sodium salts.

Particular suitable parabens according to the present invention are methylparaben, ethylparaben, butylparaben, isopropylparaben and/ or propylparaben. These parabens are e.g. commercially available as Nipagin M, Nipagin A and Nipasol M or as a mixture of parabens with phenoxyethanol as Phenonip (phenoxyethanol, methylparaben, ethylparaben, butylparaben, propylparaben, isobutylparaben) at Clariant UK Ltd. Preferably, in all embodiments of the invention the methylparaben is used in an amount ranging from 0.1 to 0.3 wt.-% and the ethyl- and propylparaben, independently of each other, are used in an amount of 0.02 to 0.1 wt.-%, based on the total weight of the composition.

Exemplary isothiazoline derived biocides according to the present invention are 5-chloro-2-methyl-4-isothiazolin-3-one (chloromethylisothiazolinone or CMIT) and 2-methyl-4-isothiazolin-3-one (methylisothiazolinone or MIT) which are the active ingredients in a 3:1 mixture (CMIT:MIT) sold commercially as Kathon™. In all embodiments of the invention the isothiazoline derived biocides are preferably used in an amount selected in the range of 0.001 to 0.05 wt.-% such as in particular in an amount selected in the range of 0.005 to 0.01 wt.-%, based on the total weight of the composition.

In all embodiments of the invention preferably the at least one preservative is selected from the group consiting of methylparaben, methylisothiazoline and phenoxyethanol as well as mixtures thereof. Even more preferably, in all embodiments of the present invention only methylparaben, methylisothiazoline and phenoxyethanol or mixtures thereof are used as preservatives.

In a particular embodiment, the compositions according to the present invention are topical compositions. The term "topical composition" as used herein refers in particular to cosmetic compositions that can be topically applied to mammalian keratinous tissue such as e.g. human skin or scalp.

The term "cosmetic composition" as used in the present application refers to cosmetic compositions as defined under the heading "Kosmetika" in Römpp Lexikon Chemie, 10th edition 1997, Georg Thieme Verlag Stuttgart, New York as well as to cosmetic compositions as disclosed in A. Domsch, "Cosmetic Compositions", Verlag für chemische Industrie (ed. H. Ziolkowsky), 4th edition, 1992.

As the topical compositions according to the present invention further comprise a physiologically acceptable medium, that is to say a medium compatible with keratinous substances, such as the skin, mucous membranes, and keratinous fibres. In particular the physiologically acceptable medium is a cosmetically acceptable carrier.

The term cosmetically acceptable carrier refers to all carriers and/or excipients and/ or diluents conventionally used in cosmetic compositions.

Preferred topical compositions according to the invention are topical compositions further comprising a cosmetically acceptable carrier. In particular the topical compositions are skin care preparations, hair care preparations, decorative preparations, and functional preparations such as particularly skin and hair care preparations.

Examples of skin care preparations are, in particular, light protective preparations, anti-ageing preparations, preparations for the treatment of photo-ageing, body oils, body lotions, body gels, treatment creams, skin protection ointments, skin powders, moisturizing preparations such as moisturizing gels, moisturizing sprays, face and/or body moisturizers, skin-tanning preparations (i.e. compositions for the artificial/sunless tanning and/or browning of human skin), for example self-tanning creams as well as skin lightening preparations.

Examples for hair care preparations are hair-washing preparations in the form of shampoos, hair conditioners, hair-care preparations such as e.g. pretreatment preparations, hair tonics, styling creams, gels such as styling gels, pomades, hair rinses, treatment packs, intensive hair treatments, hair- straightening preparations, liquid hair-setting preparations, hair foams (hair mousses) and hairsprays.

Examples of decorative preparations are, in particular, lipsticks, eye shadows, mascaras, dry and moist make-up formulations, rouges and/or powders.

Examples of functional preparations are cosmetic or pharmaceutical compositions containing active ingredients such as hormone preparations, vitamin preparations, vegetable extract preparations, anti-ageing preparations, moisturizing preparations and/or antimicrobial (antibacterial or antifungal) preparations without being limited thereto.

In a particular embodiment the topical compositions according to the invention are skin care preparations or hair care preparations.

The topical compositions according to the present invention are particular suitable for the treatment of skin and/ or scalp barrier abnormalities such as xerotic skin conditions, pruritus, itching, sensitive skin and dandruff as well as inflammatory skin and/ or scalp disorders and symptoms associated therewith.

Preferably, the topical compositions according to the invention are in the form of a suspension or dispersion in solvents or fatty substances, or alternatively in the form of an emulsion or micro emulsion (in particular of O/W- or W/O-type), PIT-emulsion, nano emulsion, multiple emulsion (e. g. O/W/O- or W/O/W-type), pickering emulsion, hydrogel, alcoholic gel, lipogel, one- or multiphase solution or vesicular dispersion or other usual forms, which can also be applied by pens, as tapes, as masks or as sprays. If the topical composition is or comprises an emulsion it can also contain one or more anionic, nonionic, cationic or amphoteric surfactant(s).

Topical compositions in accordance with the invention can be in the form of a liquid, lotion, a thickened lotion, a gel, a cream, a milk, an ointment, a paste, a powder, a make-up, or a solid tube stick and can be optionally be packaged as an aerosol and can be provided in the form of a mousse such as a aerosol mousse, a foam or a spray foam, a spray, a stick, a plaster, a cleanser, a soap, a wipe or a lyophilizate (such as the Pentapharm Dual Vial system).

The topical compositions according to the invention are preferably formulated as an oil-in-water or water-in-oil emulsion, water-in-silicone or silicone-in-water emulsion or as an aqueous serum or aqueous gel in particular as oil-in-water or water-in-oil emulsion.

The topical compositions according to the invention have a pH in the range of 3-10, preferably in the range of pH of 4-8, most preferred in the range of pH 5-7. The pH is adjusted by methods known to a person skilled in the art, e.g. by using an acid such as a hydroxy acid including glycolic acid, lactic acid, malic acid, citric acid and tartaric acid or a base such as e.g. sodium or potassium hydroxide or ammonium hydroxide as well as mixtures thereof.

Preferably, in the compositions according to the invention citric acid in an amount of at least 0.0001 wt.-%, such as e.g. in an amount of 0.01-1 wt.-%, in particular in an amount of 0.01 to 0.5 wt.-% is used for pH adjustment.

The following examples are provided to further illustrate the compositions and effects of the present invention. These examples are illustrative only and are not intended to limit the scope of the invention in any way.

### Example 1: Antimicrobial Efficacy

The antimicrobial activity was assessed by determination of the Minimum Inhibitory Concentration (MIC) using the agar dilution method according to DIN 58 940 at the Labor L+SAG, D- Bad Bocklet-Grossenbrach.

Culture plates with a diameter of 5.5 cm were prepared with fresh, kept liquid (50°C) Mueller-Hinton Agar (double concentrated, Merck 1.05437)/ aqua purificata comprising the compound/ compound mixtures in the concentrations indicated in Table 1 according to standard methods. After solidifying and drying (about 1h at 36°C), the culture plates were inoculated with 1 µl of a suspension of the respective microorganism with the colony forming unit as outlined in table 1. The inoculated plates were then incubated at 36°C. The MIC's (i.e. the concentrations where still no growth could be observed) were determined after 18h for E. coli, P. aeruginosa and S. aureus; after 48h for P. acnes and after 78 h for A. brasiliensis and C. albicans. Per sample two culture plates were prepared. As control incubated and non-incubated cultures were used. The incubated control samples showed and the non-incubated control samples did not show microbial growth.

**Table 1: Test series**

| **Sample** | **Final concentration in [mg/ml]** | | | | | | | | | |
|---|---|---|---|---|---|---|---|---|---|---|
| pHBA | 40 | 26.67 | 17.78 | 11.85 | 7.90 | 5.27 | 3.51 | 2.34 | 1.56 | 1.04 |
| PE | 10 | 6.67 | 4.44 | 2.96 | 1.98 | 1.32 | 0.88 | 0.59 | 0.39 | 0.26 |
| MI | 0.5 | 0.33 | 0.22 | 0.15 | 0.1 | 0.07 | 0.04 | 0.03 | 0.02 | 0.01 |
| MP | 2.0 | 1.33 | 0.89 | 0.59 | 0.39 | 0.26 | 0.18 | 0.12 | 0.08 | 0.05 |
| pHBA | 40 | 26.67 | 17.78 | 11.85 | 7.90 | 5.27 | 3.51 | 2.34 | 1.56 | 1.04 |
| PE | 10 | 6.67 | 4.44 | 2.96 | 1.98 | 1.32 | 0.88 | 0.59 | 0.39 | 0.26 |
| pHBA | 25 | 16.7 | 11.1 | 7.4 | 4.9 | 3.3 | 2.2 | 1.5 | 0.98 | 0.65 |
| MI | 0.5 | 0.33 | 0.22 | 0.15 | 0.1 | 0.07 | 0.04 | 0.03 | 0.02 | 0.01 |
| pHBA | 8 | 5.13 | 3.56 | 2.4 | 1.58 | 1.05 | 0.5 | 0.47 | 0.31 | 0.21 |
| MP | 2 | 1.3 | 0.89 | 0.59 | 0.39 | 0.26 | 0.18 | 0.12 | 0.08 | 0.05 |

- pHBA:: p-Hydroxybenzylamine
- PE:: Phenoxyethanol
- MI:: Methylisothiazolinone
- MP:: Methylparabene

The suspensions of the microorganisms No. 1-4 were prepared by homogeneous suspension of 3-5 colonies of the same morphology in physiological NaCl-solution. The suspensions were adjusted such, that the haze corresponded to the Mac Farland standard 1.0 (about 3,0 * 10⁸ KBE*/ml). For the preparation of the suspensions of the microorganisms 5 and 6, the microorganisms have been taken with a sterile swab and diluted in an amount of physiological NaCl solution such, that the haze corresponded to the Mac Farland standard 1.0. Afterwards the suspensions of the microorganisms 1 and 3-6 have been further diluted with physiological NaCl-solution in a ratio of 1:10. The colony count was determined using a spiral plater system (see table 2).

**Table 2**

| No | Culture | | Susp. 1 | Susp. 2 |
|---|---|---|---|---|
| | | | KBE*/ml | |
| 1 | Escherichia coli (E. coli) | ATCC 8739 | 2.4*10⁷ | 1.9*10⁷ |
| 2 | Pseudomonas aeruginosa (P. aeruginosa) | ATCC 9027 | 2.7*10⁸ | 2.1*10⁸ |
| 3 | Staphylococcus aureus (S. aureus) | ATCC 6538 | 3.0*10⁷ | 1.8*10⁷ |
| 4 | Propionibacterium acnes (P. acnes) | ATCC 11828 | 2.3*10⁷ | 2.6*10⁷ |
| 5 | Aspergillus brasiliensis (A. brasiliensis) | ATCC 16404 | 2.9*10⁷ | 3.0*10⁷ |
| 6 | Candida albicans (C. albicans) | ATCC 10231 | 2.3*10⁷ | 2.0*10⁷ |

| | | | | |
|---|---|---|---|---|
| *Colony forming units | | | | |

The results are presented in the tables 3-6 below. The synergistic effect is shown by calculation of the ratio [MIC (single)/ (MIC (mixture)]. A value of > 1 indicates a synergistic effect.

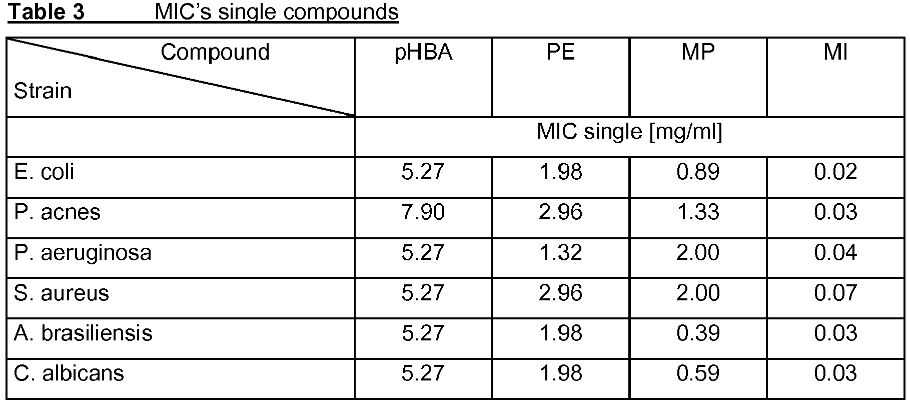

**Table 4 MIC's mixture pHBA/ PE**

| Strain | Compound | MIC mix [mg/ml] | ratio [MIC (single)/ (MIC (mixture)] |
|---|---|---|---|
| E. coli | pHBA | 3.51 | 1.50 |
| | PE | 0.88 | 2.25 |
| P. acnes | pHBA | 5.27 | 1.50 |
| | PE | 1.32 | 2.24 |
| P. aeruginosa | pHBA | 3.51 | 1.50 |
| | PE | 0.88 | 1.50 |
| S. aureus | pHBA | 7.90 | 0.67 |
| | PE | 1.98 | 1.49 |
| A. brasiliensis | pHBA | 2.34 | 2.25 |
| | PE | 0.59 | 3.36 |
| C. albicans | pHBA | 2.34 | 2.25 |
| | PE | 0.59 | 3.36 |

**Table 5 MIC's mixture pHBA/ MP**

| Strain | Compound | MIC mix [mg/ml] | ratio [MIC (single)/ (MIC (mixture)] |
|---|---|---|---|
| E. coli | pHBA | 1.58 | 3.34 |
| | MP | 0.39 | 2.28 |
| P. acnes | pHBA | 3.56 | 2.22 |
| | MP | 0.89 | 1.49 |
| P. aeruginosa | pHBA | 2.40 | 2.20 |
| | MP | 0.59 | 3.39 |
| S. aureus | pHBA | 3.56 | 1.48 |
| | MP | 0.89 | 2.25 |
| A. brasiliensis | pHBA | 1.05 | 5.02 |
| | MP | 0.26 | 1.50 |
| C. albicans | pHBA | 1.58 | 3.34 |
| | MP | 0.39 | 1.51 |

**Table 6 MIC's mixture pHBA/ MI**

| Strain | Compound | MIC mix [mg/ml] | ratio [MIC (single)/ (MIC (mixture)] |
|---|---|---|---|
| E. coli | pHBA | 0.98 | 5.38 |
| | MI | 0.02 | 1.00 |
| P. acnes | pHBA | 0.65 | 12.15 |
| | MP | <0.01 | >100 |
| P. aeruginosa | pHBA | 0.65 | 8.11 |
| | MI | <0.01 | >100 |
| S. aureus | pHBA | 2.20 | 2.4 |
| | MI | 0.04 | 1.75 |
| A. brasiliensis | pHBA | 0.98 | 5.38 |
| | MI | 0.02 | 1.50 |
| C. albicans | pHBA | 0.98 | 5.38 |
| | MI | 0.02 | 1.50 |

### Example 2: O/W Foundation

| *Ingredients* | *INCI* | *wt. %* |
|---|---|---|
| Deionised Water | Aqua | Ad 100 |
| Glycerin | Glycerin | 2.00 |
| Triethanolamine | Triethanolamine | 0.80 |
| Paratexin | Methylparaben EP | 0.20 |
| Keltrol | Xanthan Gum | 0.30 |
| p-Hydroxybenzylamine | | 1.50 |
| Titanium dioxide | C.I. 77891 | 4.57 |
| SunCROMA yellow iron oxide | C.I. 77492 | 0.30 |
| SunCROMA red iron oxide | C.I. 77491 | 0.13 |
| SunCROMA black iron oxide | C.I. 77499 | 0.20 |
| DC 556 | Phenyl Trimethicone | 3.60 |
| Stearic Acid | Stearic Acid | 1.4 |
| Cetyl Alcohol | Cetyl Alcohol | 3.0 |
| Paratexin P | Propylparaben EP | 0.1 |

### Example 3: Alcohol Free Facial Tonic

| *Ingredients* | *INCI* | *wt. %* |
|---|---|---|
| Polysorbate 20 | Polysorbate 20 | 2.00 |
| ALPAFLOR CALENDULA AO | Calendula Officinalis Extract, Glycerin, Water | 0.80 |
| ALPAFLOR BUDDLEJA AO | Buddleja Davidii Extract, Glycerin, Water | 0.80 |
| Arlasilk Phospholipd CDM | Sodium Coco PG-Dimonium Chloride Phosphate | 0.50 |
| Fragrance | Parfum | 0.10 |
| Deionised Water | Aqua | Ad 100 |
| Citric Acid | Citric Acid | 0.01 |
| p-Hydroxybenzylamine | | 1.00 |
| Paratexin FRP | Ethylparaben, Butylparaben, Propylparaben, Isobutylparaben | 0.10 |

### Example 4: W/O Cream

| *Ingredients* | *INCI* | *wt. %* |
|---|---|---|
| Cremophor WO-7 | PEG-7 Hydrogenated Castor Oil | 2.50 |
| Elfacos ST-9 | PEG-45/Dodecyl Glycol Copolymer | 2.00 |
| Cirebelle 303 | Synthetic Wax | 5.00 |
| Cirebelle 109L | Synthetic Wax | 7.20 |
| Miglyol 818 | Caprylic/Capric/Linoleic Triglyceride | 5.00 |
| Eutanol G | Octyldodecanol | 7.50 |
| Cetiol OE | Dicaprylyl Ether | 9.20 |
| Deionised Water | Aqua | Ad 100 |
| Glycerin | Glycerin | 5.00 |
| Propylene Glycol | Propylene Glycol | 2.00 |
| Euxyl PE 9010 | Phenoxyethanol and Ethylhexylglycerin | 0.80 |
| p-Hydroxybenzylamine | | 1.50 |

### Example 5: Soothing Gel

| *Ingredients* | *INCI* | *wt. %* |
|---|---|---|
| Deionised Water | Aqua | Ad 100 |
| Keltrol CG RD | Xanthan Gum | 0.50 |
| Sodium Benzoate | Sodium Benzoate | 0.20 |
| Potassium Sorbate | Potassium Sorbate | 0.25 |
| ALPAFLOR MARRABIUM AO | Glycerin, Aqua, Marrubium Vulgare, Sodium Benzoate, Potassium Sorbate | 3.00 |
| p-Hydroxybenzylamine | | 3.0 |

### Example 6: O/W Lotion

| *Ingredients* | *INCI* | *wt. %* |
|---|---|---|
| Deionised Water | Aqua | Ad 100 |
| Menthol | Menthol | 0.10 |
| Keltrol CG SFT | Xanthan Gum | 1.25 |
| Ceralution ES | Ceteareth-25, Di Sodium Ethylene Dicocamide PEG-15 Disulfate | 2.00 |
| Isofol 20 | Octyldodecanol | 5.00 |
| Paratexin EC5 | Benzoic Acid Benzyl Alcohol, Dehydroacetic Acid, Sorbic Acid | 1.00 |
| p-Hydroxybenzylamine | | 1.50 |

### Example 7: Facial Cleansing Gel

| *Ingredients* | *INCI* | *wt. %* |
|---|---|---|
| Deionised Water | Aqua | Ad 100 |
| Carbopol AQUA SF-1 Polymer | Acrylates Copolymer | 7.50 |
| Texapon NSO-BZ | Sodium Laureth Sulfate | 41.00 |
| Miranol Ultra C 32 | Sodium Cocoamphoacetate | 5.00 |
| Hostapon CLG | Sodium Lauroyl Glutamate | 4.50 |
| Jaguar C 162 | Hydroxypropyl Guar Hydroxypropyltrimonium Chloride | 1.00 |
| p-Hydroxybenzylamine | | 0.50 |
| Euxyl K 300 | Phenoxyethanol & Methylparaben & Propylparaben & Ethylparaben & Butylparaben & Isobutylparaben | 0.80 |
| ALPAFLOR MALVA AO | Glycerin, Aqua, Malva Sylvestris (Mallow) Flower | 2.00 |
| | Extract, Potassium Sorbate, Sodium Benzoate | |
| Parfum Limette | Fragrance | q.s. |
| FD&C Yellow 5 | Cl 19140 | 0.50 |
| Frescolat Plus | Menthyl Lactate, Menthol | 0.20 |
| Dehyton AB-30 | Coco Betaine | 2.00 |
| Rewoderm LI S 80 | PEG-200 Hydrogenated Glyceryl Palmate & PEG-7 Glyceryl Cocoate | 1.00 |
| Citric Acid | Citric Acid | q.s. |

### Example 8: Leave-on Hair and Scalp Conditioner

| *Ingredients* | *INCI* | *wt. %* |
|---|---|---|
| Deionised Water | Aqua | Ad 100 |
| Ethanol DEB 96 | Alcohol denat. | 30.00 |
| PVP/VA Copolymer | PVP/VA Copolymer | 2.50 |
| Euxyl K-300 | Phenoxyethanol, Methylparaben, Butylparaben, Ethylparaben, Propylparaben, Isobutylparaben | 0.80 |
| Protachem HCO-40 | PEG-40 Hydrogenated Castor Oil | 0.50 |
| Fragrance ADAM | Parfum | 0.10 |
| Triethanolamine 99% | Triethanolamine | 0.01 |
| FD & C Yellow No 5 (0.5% Solution) | CI 19140, Aqua | 0.10 |
| FD & C Blue No 1 (0.5% Solution) | CI 42090, Aqua | 0.10 |
| p-Hydroxybenzylamine | | 0.5 |

### Example 9: Anti Dandruff Shampoo

| *INCI Nomenclature* | *wt. %* |
|---|---|
| Aqua (water) | Ad 100 |
| Ammonium laureth sulfate | 10.00 |
| Ammonium lauryl sulfate | 5.00 |
| Glycol distearate | 1.00 |
| Dimethicone | 1.00 |
| Cetyl alcohol | 0.50 |
| Cocamide MEA | 3.00 |
| p-Hydroxybenzylamine | 2.50 |
| ZPT | 0.50 |
| Guar hydroxypropyltrimonium chloride | 0.20 |
| Hydrogenated polydecene | 1.00 |
| Polyquaternium 10 | 0.30 |
| PEG 7m | 0.50 |
| Trimethylpropane tricaprylate/ tricaprate | 1.00 |
| Preservative | q.s. |
| Fragrance | 0.30 |
| E 104, E 110, E 132 | 0.02 |

### Example 10:Clear Anti Dandruff Shampoo with plant extracts

| *INCI Nomenclature* | *wt. %* |
|---|---|
| Aqua (water) | Ad 100 |
| Sodium laureth sulfate | 10.00 |
| Lauryl glucoside | 6.00 |
| Cocamidopropyl betaine, | 2.00 |
| Propylene glycol | 2.00 |
| Perfume oil | 1.25 |
| Sodium citrate | 0.25 |
| Sodium benzoate | 0.20 |
| Panthenol | 1.00 |
| Sodium formate | 0.20 |
| Polyquaternium-10 | 0.20 |
| Hydroxypropyl guar hydroxypropyltrimonium chloride | 0.05 |
| p-Hydroxybenzylamine | 5.00 |
| PEG-35 castor oil | 1.00 |
| Maris sal | 1.25 |
| Polysorbate 20 | 1.00 |
| Tocopheryl acetate | 0.20 |
| Prunus armeniaca | 0.20 |
| Echinacea purpurea | 0.05 |
| Retinyl palmitate | 0.05 |
| Tocopherol | 0.05 |
| Linoleic acid | 0.20 |
| Preservative | 1.00 |
| Cl77891 | 0.02 |

### Example 11: Rinse-off Hair and Scalp Conditioner

| *INCI Nomenclature* | *wt. %* |
|---|---|
| Aqua (water) | Ad 100 |
| Stearyl alcohol | 2.50 |
| Cetyl alcohol | 2.50 |
| Behentrimonium chloride | 1.30 |
| Dimethicone | 2.00 |
| p-Hydroxybenzylamine | 1.50 |
| Fragrance | 0.50 |
| Butylene glycol | 2.00 |
| Methyl parabene | 0.30 |

## Claims

1. A composition comprising of p-hydroxybenzylamine or a salt thereof and at least one preservative selected from the group consisting of parabens, chloromethylisothiazolinone, methylisothiazolinone or phenoxyethanol as well as mixtures thereof.

2. The composition according to claim 1, wherein the ratio (w/w) of p-hydroxybenzylamine or a salt thereof to the total amount of preservative(s) is >1.

3. The composition according to claim 2, wherein of ratio (w/w) of p-hydroxybenzylamine or a salt thereof to the total amount of preservative(s) is selected in the range of 2:1 to 100:1.

4. The compositons according to any one of claims 1 to 3, wherein the amount of p-hydroxybenzylamine or a salt thereof is selected in the range of 0.001 to 10 wt.-% based on the total weight of the composition.

5. The composition according to any one of claim 1 to 4, wherein the amount of the at least one preservative is selected in the range of 0.001 to 5 wt.-%, based on the total weight of the composition.

6. The composition according to any one of claims 1 to 5, wherein p-hydroxybenzylamine is used in the form of its hydrochloride.

7. The composition according to any one of claims 1 to 6, wherein the at least one preservative is selected from the group consisting of methylparaben, methylisothiazoline and phenoxyethanol or mixtures thereof.

8. The composition according to any one of claims 1 to 7, wherein the composition is a topical composition further comprising a cosmetically acceptable carrier.

9. The topical composition according to claim 8, wherein the composition is a skin or hair care preparation.

10. Use of p-hydroxybenzylamine or a salt thereof for increasing the antimicrobial activity of at least one preservative selected from the group consisting of parabens, chloromethylisothiazolinone, methylisothiazolinone or phenoxyethanol as well as mixtures thereof.

11. Use according to claim 10, wherein the antimicrobial activity is an antifungal and/ or antibacterial activity.

12. Non-therapeutic method for killing and/ or inhibiting growth of microbial cells, in particular fungal and/ or bacterial cells, said method comprising contacting said microbial cells with a mixture consisting of p-hydroxybenzylamine or a salt thereof and at least one preservative selected from the group consisting of parabens, chloromethylisothiazolinone, methylisothiazolinone or phenoxyethanol as well as mixtures thereof.

13. Method according to claim 12, wherein the microbial cells are fungal and/ or bacterial cells.

14. Method according to claim 13, wherein the fungal and/ or bacterial cells are Escherichia Coli, Pseudomonas aeruginosa, Staphylococcus aureus, Propionibacterium acnes, Aspergillus brasiliensis and/ or Candida albicans.

## Patentansprüche

1. Zusammensetzung umfassend p-Hydroxybenzylamin oder ein Salz davon und mindestens ein Konservierungsmittel, ausgewählt aus der Gruppe bestehend aus Parabenen, Chlormethylisothiazolinon, Methylisothiazolinon oder Phenoxyethanol sowie Mischungen davon.

2. Zusammensetzung nach Anspruch 1, wobei das Verhältnis (w/w) von p-Hydroxybenzylamin oder einem Salz davon zu der Gesamtmenge an Konservierungsmittel(n) >1 beträgt.

3. Zusammensetzung nach Anspruch 2, wobei ein Verhältnis (w/w) von p-Hydroxybenzylamin oder einem Salz davon zu der Gesamtmenge an Konservierungsmittel(n) im Bereich von 2:1 bis 100:1 ausgewählt ist.

4. Zusammensetzungen nach einem der Ansprüche 1 bis 3, wobei die Menge an p-Hydroxybenzylamin oder einem Salz davon im Bereich von 0,001 bis 10 Gew.-% in Bezug auf das Gesamtgewicht der Zusammensetzung ausgewählt ist.

5. Zusammensetzung nach einem der Ansprüche 1 bis 4, wobei die Menge des mindestens einen Konservierungsmittels im Bereich von 0,001 bis 5 Gew.-% in Bezug auf das Gesamtgewicht der Zusammensetzung ausgewählt ist.

6. Zusammensetzung nach einem der Ansprüche 1 bis 5, wobei p-Hydroxybenzylamin in Form seines Hydrochlorids eingesetzt wird.

7. Zusammensetzung nach einem der Ansprüche 1 bis 6, wobei das mindestens eine Konservierungsmittel aus der Gruppe bestehend aus Methylparaben, Methylisothiazolin und Phenoxyethanol oder Mischungen davon ausgewählt ist.

8. Zusammensetzung nach einem der Ansprüche 1 bis 7, wobei es sich bei der Zusammensetzung um eine topische Zusammensetzung, die weiterhin einen kosmetisch unbedenklichen Träger umfasst, handelt.

9. Zusammensetzung nach Anspruch 8, wobei es sich bei der Zusammensetzung um ein Haut- oder Haarpflegepräparat handelt.

10. Verwendung von p-Hydroxybenzylamin oder einem Salz davon zum Erhöhen der antimikrobiellen Aktivität von mindestens einem Konservierungsmittel, ausgewählt aus der Gruppe bestehend aus Parabenen, Chlormethylisothiazolinon, Methylisothiazolinon oder Phenoxyethanol sowie Mischungen davon.

11. Verwendung nach Anspruch 10, wobei es sich bei der antimikrobiellen Aktivität um eine antifungale und/oder antibakterielle Aktivität handelt.

12. Nichttherapeutisches Verfahren zum Abtöten und/oder Hemmen des Wachstums von Mikroorganismenzellen, insbesondere Pilz- und/oder Bakterienzellen, wobei das Verfahren das Inkontaktbringen der Mikroorganismenzellen mit einer Mischung bestehend aus p-Hydroxybenzylamin oder einem Salz davon und mindestens einem Konservierungsmittel, ausgewählt aus der Gruppe bestehend aus Parabenen, Chlormethylisothiazolinon, Methylisothiazolinon oder Phenoxyethanol sowie Mischungen davon, umfasst.

13. Verfahren nach Anspruch 12, wobei es sich bei den Mikroorganismenzellen um Pilz- und/oder Bakterienzellen handelt.

14. Verfahren nach Anspruch 13, wobei es sich bei den Pilz- und/oder Bakterienzellen um Escherichia Coli, Pseudomonas aeruginosa, Staphylococcus aureus, Propionibacterium acnes, Aspergillus brasiliensis und/oder Candida albicans handelt.

## Revendications

1. Composition comprenant de la p-hydroxybenzylamine ou un sel de celle-ci et au moins un conservateur choisi dans le groupe constitué par les parabènes, la chlorométhylisothiazolinone, la méthylisothiazolinone ou le phénoxyéthanol, ainsi que des mélanges de ceux-ci.

2. Composition selon la revendication 1, dans laquelle le rapport (p/p) de la p-hydroxybenzylamine ou d'un sel de celle-ci à la quantité totale du ou des conservateurs est > 1.

3. Composition selon la revendication 2, dans laquelle le rapport (p/p) de la p-hydroxybenzylamine ou d'un sel de celle-ci à la quantité totale du ou des conservateurs est choisi dans la plage allant de 2:1 à 100:1.

4. Compositions selon l'une quelconque des revendications 1 à 3, dans laquelle la quantité de p-hydroxybenzylamine ou d'un sel de celle-ci est sélectionnée dans la plage allant de 0,001 à 10% en poids sur la base du poids total de la composition.

5. Composition selon l'une quelconque des revendications 1 à 4, dans laquelle la quantité du au moins un conservateur est sélectionnée dans la plage allant de 0,001 à 5% en poids sur la base du poids total de la composition.

6. Composition selon l'une quelconque des revendications 1 à 5, dans laquelle la p-hydroxybenzylamine est utilisée sous la forme de son chlorhydrate.

7. Composition selon l'une quelconque des revendications 1 à 6, dans laquelle le au moins un conservateur est choisi dans le groupe constitué par le p-hydroxybenzoate de méthyle, la méthylisothiazoline et le phénoxyéthanol, ou des mélanges de ceux-ci.

8. Composition selon l'une quelconque des revendications 1 à 7, où la composition est une composition topique comprenant en outre un véhicule cosmétiquement acceptable.

9. Composition topique selon la revendication 8, où la composition est une préparation de soin de la peau ou des cheveux.

10. Utilisation de p-hydroxybenzylamine ou d'un sel de celle-ci afin d'augmenter l'activité antimicrobienne d'au moins un conservateur choisi dans le groupe constitué par les parabènes, la chlorométhylisothiazolinone, la méthylisothiazolinone ou le phénoxyéthanol, ainsi que des mélanges de ceux-ci.

11. Utilisation selon la revendication 10, dans laquelle l'activité antimicrobienne est une activité antifongique et/ou antibactérienne.

12. Méthode non thérapeutique de destruction et/ou d'inhibition de cellules microbiennes, en particulier de cellules fongiques et/ou bactériennes, ladite méthode comprenant la mise en contact desdites cellules microbiennes avec un mélange constitué de p-hydroxybenzylamine ou d'un sel de celle-ci et d'au moins un conservateur choisi dans le groupe constitué par les parabènes, la chlorométhylisothiazolinone, la méthylisothiazolinone ou le phénoxyéthanol, ainsi que des mélanges de ceux-ci.

13. Méthode selon la revendication 12, dans laquelle les cellules microbiennes sont des cellules fongiques et/ou bactériennes.

14. Méthode selon la revendication 13 , dans laquelle les cellules fongiques et/ou bactériennes sont *Escherichia coli, Pseudomonas aeruginosa, Staphylococcus aureus, Propionibacterium acnes, Aspergillus brasiliensis* et/ou *Candida albicans.*
